# EUROPÄISCHE PATENTANMELDUNG

(11) **EP 0 621 020 A1**
(43) Veröffentlichungstag der Anmeldung: **26.10.1994**
(21) Anmeldenummer: 94810165.4
(22) Anmeldetag: 17.03.1994
(51) Int. Cl.: A61F 2/44

(54) **Zwischenwirbelprothese und Verfahren zum Implantieren einer derartigen Prothese**

(30) Priorität: 21.04.1993 EP 93810291
(71) Anmelder: SULZER Medizinaltechnik AG, CH-8404 Winterthur (CH); PROTEK AG, CH-3110 Münsingen-Bern (CH)
(72) Erfinder: Baumgartner, Walter, CH-9500 Wil (CH)
(74) Vertreter: Trieblnig, Adolf

(57) **Zusammenfassung**

Als Ersatz für einen nicht mehr tragfähigen Teil des Kernbereichs einer Bandscheibe (3), ist ein Implantat aus mehreren Stützkörpern (7) vorgesehen, welche aus einem elastischen Kunststoff gefertigt sind. Die Stützkörper (7) werden nacheinander mittels einer einen äusseren Ringbereich (4) der Bandscheibe (3) durchsetzenden Sonde (6) in einen im Kernbereich ausgebildeten zentralen Hohlraum (5) eingeführt, bis dieser ausgefüllt ist. Beim Stopfen des Hohlraums (5) mit den Füllkörpern (7) legen sich diese an die Begrenzungswände des Ringbereichs (4) und gegeneinander an und werden unter Belastung elastisch deformiert. Entsprechend wird ein universell einsetzbares und an Hohlräume (5) beliebiger Form anpassbares Implantat erzielt, welches eine relativ kompakte, elastische Tragstruktur bildet.

## Beschreibung

Die Erfindung betrifft eine Zwischenwirbelprothese gemäss dem Oberbegriff des Patentanspruchs 1 sowie ein Verfahren zum Implantieren einer derartigen Prothese.

Bekannte Zwischenwirbelprothesen der genannten Art enthalten Implantate für Bandscheiben, die über eine rohrförmige Sonde durch den äusseren Ring (annulus fibrosus) der Bandscheibe in deren Kernbereich (nucleus pulposus) einführbar sind, um in Richtung der Hauptlast eine Tragwirkung zu erzielen. So zeigt die EP-A- 0 453 393 einen in den Kernbereich der Bandscheibe einführbaren Hohlkörper, der in Form einer Spirale aufwickelbar ist und der in aufgewickeltem Zustand mit einer inkompressiblen Flüssigkeit auffüllbar ist. Vor dem Einsetzen eines solchen Implantates muss der nicht mehr tragfähige Kernbereich der Bandscheibe durch die hohle Sonde hindurch mit Hilfswerkzeugen ausgeräumt werden, um das nicht mehr tragfähige Material durch das Implantat zu ersetzen. Da der Operateur von bestimmten Implantatsgrössen ausgehen muss, ist er gezwungen, einen passenden Hohlraum im Kernbereich der Bandscheibe herzustellen. Das bekannte Implantat verlangt eine relativ aufwendige Ausführung des Hohlkörpers, um eine für eine optimale Funktion des Implantats erforderliche, bleibende Dichtigkeit gegen Austreten von Flüssigkeit zu gewährleisten.

Diesen Nachteilen soll die Erfindung entgegenwirken.

Sie hat die Aufgabe, ein universelles, einfach applizierbares Implantat zu schaffen, welches für die verschiedensten, beliebig geformten Hohlräume als Tragkörper anwendbar ist.

Diese Aufgabe wird erfindungsgemäss dadurch gelöst, dass das Implantat mindestens drei in den zentralen Hohlraum einfüllbare und in diesem positionierbare, elastisch verformbare Stützkörper enthält.

Ein Vorteil der Erfindung besteht darin, dass der Operateur bei der Schaffung des Hohlraumes nur das nicht mehr tragfähige Material des Nucleus entfernen muss und dass sich die einfüllbare Menge der Stützkörper zwangsläufig beim Einfüllen der Stützkörper ergibt. Das Implantat aus diesen im Abstand voneinander oder einander berührend positionierbaren Stützkörpern, die aus einem beliebigen körperverträglichen, elastischen Material bestehen können, ist somit individuell für jede Bandscheibe geeignet. Beim Stopfen des zentralen Hohlraumes mit den Stützkörpern legen sich diese an die Begrenzungswände an, so dass eine allseitige Uebertragung der Druckkräfte und eine optimale Verteilung im zentralen Hohlraum erzielbar ist. Bei Belastung werden die Stützkörper elastisch deformiert, und die in Richtung der Körperachse wirkenden Druckkräfte werden in Ringspannungen im anulus fibrosus umgesetzt.

Ein erfindungsgemässes Verfahren zum Implantieren einer derartigen Zwischenwirbelprothese ist Gegenstand des Patentanspruchs 15.

Ausgestaltungen des Erfindungsgegenstandes sind in den abhängigen Patentansprüchen angegeben.

Nach einer bevorzugten Ausführungsform nach der Erfindung können die Stützkörper aus einem elastischen Kunststoff gefertigt sein. Entsprechend kann auf einfache Weise ein mit geringem Aufwand herstellbares, dauerhaft formstabiles Implantat aus einem geeigneten körperverträglichen Kunststoffmaterial erzielt werden.

Die Stützkörper können vorzugsweise in Form von Rotationskörpern ausgeführt sein, welche eine optimale, gleichmässige Uebertragung der Druckkräfte ermöglichen. In dieser Hinsicht sind insbesondere kugelförmige Ausführungen von Vorteil.

Durch die Verwendung von Stützkörpern mit unterschiedlichen Abmessungen kann die Packungsdichte vergrössert werden. Durch das Anbringen von Kanälen in den Stützkörpern können Hohlformen entstehen, die im Gegensatz zu Vollkörpern eine definierte, grössere elastische Deformation zulassen. Um die Steifigkeit des Implantates zu steuern, können auch Vollkörper und Hohlkörper als Gemisch eingeführt werden. Für die Stützkörper selbst ergibt sich eine Vielzahl von Formen, die von der nicht orientierten Kugelform über linsenförmige, bohnenförmige bis zu länglichen, zylinderartigen, wurstförmigen Körpern geht.

Mehrere Stützkörper können in Ketten mit einem schnurartigen flexiblen Träger verbunden sein, wobei der Abstand zwischen zwei Stützkörpern vorteilhafterweise mindestens dem Durchmesser eines der Stützkörper entspricht, um Umlenkungen von 180° beim Stopfen der Stützkörper zu ermöglichen. Es sind auch einstückige Ketten aus dem gleichen Material denkbar, bei denen die flexiblen Zwischenstücke entsprechend dünn ausgeführt sind. Ebenso ist es denkbar, Stützkörper mit einem flexiblen Schlauch auf Abstand zueinander zu halten. Die Kettenform der Stützkörper hat einerseits den Vorteil, dass ein Stützkörper in einer Kette den zentralen Hohlraum nicht ohne weiteres durch eine Oeffnung verlassen kann und andererseits, dass während des Einfüllens der Vorgang ohne grossen Zeitverlust durch Rückziehen der Kette rückgängig gemacht werden kann. Eine ähnliche Rückhaltewirkung ist an Einzelstützkörpern mit elastisch verformbaren Spreizelementen erreichbar, welche beim Einführen durch die Sonde in Längsrichtung deformiert sind und beim Eintritt in den zentralen Hohlraum zurückfedern und einen grösseren Querschnitt einnehmen.

Eine weitere Möglichkeit des Ablegens von Stützkörpern in einer bestimmten Orientierung besteht darin, den Querschnitt der Sonde auf Führungsflächen der Stützkörper anzupassen und die Stützkörper gezielt - etwa wie ein Insekt beim Eierlegen - an bestimmten Orten des zentralen Hohlraumes abzulegen, indem der Stützkörper in der Sonde geführt ist und mit einem Stössel ausgestossen wird. Ein linsenförmiger Körper kann z.B. so abgelegt werden, dass seine Flachseiten gegen die benachbarten Wirbel gerichtet sind.

Wenn die Stützkörper einen Lageanzeiger, etwa in Form eines Einschlusses aus einem unter Röntgenbeobachtung sichtbaren Material, wie z.B. Tantal, aufweisen, sind das Ablegen und spätere Lageänderungen kontrollierbar.

Als Kunststoff für solche Stützkörper ist z.B. Polyurethan geeignet. Die Stützkörper können auch aus einem anderen Material, z.B. einem Hydrogel, gefertigt sein. Entsprechende Stützkörper können auch aus einem geeigneten Schaumstoff ausgeführt sein. Es ist auch eine Ausführung vorstellbar, bei der die Stützkörper je durch einen kokonartigen Wickel aus einem Kunststoffaden oder einem Metallfaden gebildet sind.

Um die Sicherheit für das Einfüllen und Beeinanderbleiben der Stützkörper zu erhöhen, kann vor dem Einfüllen der Stützkörper ein Sack aus Kunststoffgewebe oder Kunststoffolie durch die Sonde in den zentralen Hohlraum eingebracht werden, wobei die Oeffnung des Sacks ausserhalb der Sonde verbleibt. Die Stützkörper werden nun durch die Oeffnung dieses Sackes eingefüllt, welche sich an der Sonde abstützt. Wenn der zentrale Hohlraum und der Sack mit Stützkörpern gestopft sind, kann der Sack mit einer Klammer oder einem Draht abgebunden werden, um ein Austreten von Stützkörpern zu verhindern.

Weitere Merkmale und Einzelheiten ergeben sich aus der folgenden Beschreibung von in der Zeichnung schematisch dargestellten Ausführungsbeispielen der Erfindung, in Verbindung mit den Ansprüchen. Es zeigen:
- Fig. 1: einen Wirbelkörper in einer Draufsicht mit einem Querschnitt durch eine Bandscheibe, die ein Implantat aus Stützkörpern enthält, welche über eine Sonde zuführbar sind;
- Fig. 2: die Bandscheibe in einem Längsschnitt entsprechend der Linie II-II in Fig. 1;
- Fig. 3: einen Querschnitt durch eine Bandscheibe, mit einem Implantat in einer abgewandelten Ausführungsform;
- Fig. 3a: einen Querschnitt durch einen Sack mit Stützkörpern;
- Fig. 4a - 4e: verschiedene Ausführungsformen von erfindungsgemäss ausgebildeten Stützkörpern.
- Fig. 5: den Stützkörper nach Fig. 4e in einer Stellung beim Einführen durch eine in einem Teillängsschnitt dargestellte Sonde;
- Fig. 6, 7 und 8: Implantate aus mehreren miteinander verbundenen Stützkörpern, je in einer anderen Ausführungsform.

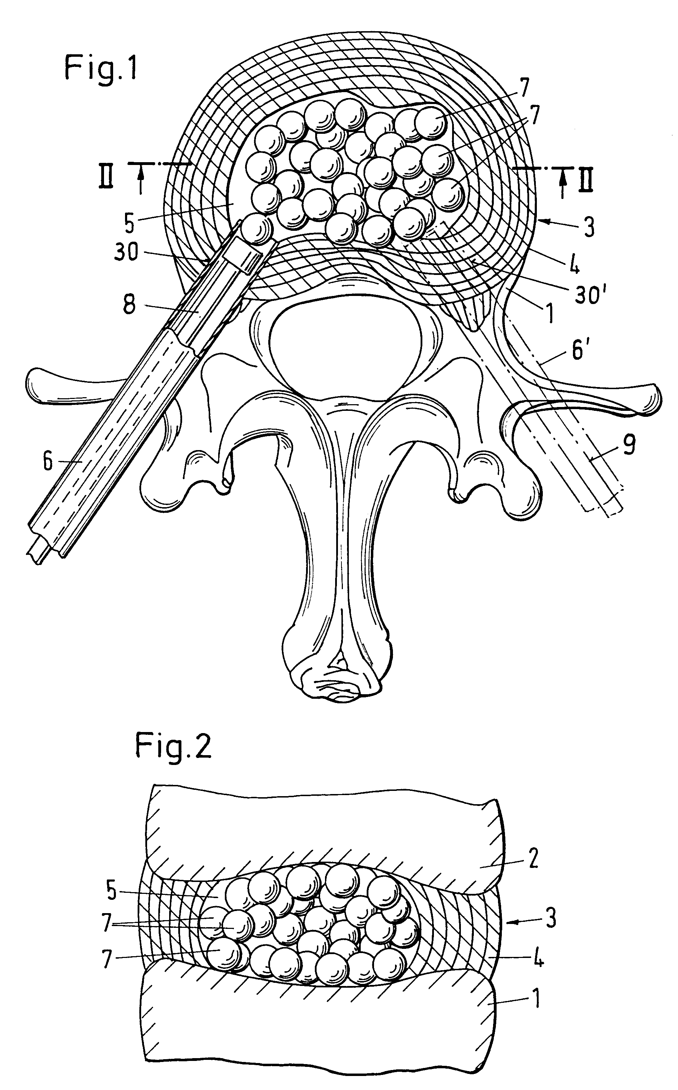

Entsprechend der Darstellung nach den Fig. 1 und 2 weist eine zwischen zwei Wirbelkörpern 1 und 2 befindliche Bandscheibe 3 einen intakten äusseren Ringbereich 4 aus natürlichem Gewebe auf, der einen zentralen Kernbereich umschliesst. Im Kernbereich ist ein Hohlraum 5 ausgebildet, der vorgängig durch Entfernen des beschädigten, nicht mehr tragfähigen Bandscheibenkerns - oder eines Teils davon - und gegebenenfalls schadhafter Teile des äusseren Ringbereichs 4 geschaffen wurde. Die Bildung des Hohlraums 5 und die Entfernung der nicht mehr tragfähigen Gewebeteile erfolgt in bekannter Weise durch einen rohrförmigen Führungsteil, darstellungsgemäss in Form einer Sonde 6, welche, wie z.B. in der eingangs erwähnten EP-A-O 453 393 beschrieben, durch einen relativ geringfügigen Eingriff von dorsal her zwischen den Wirbelkörpern 1 und 2 hindurch, eine Oeffnung 30 im äusseren Ringbereich 4 durchsetzend, in den Kernbereich der Bandscheibe 3 eingeführt wird. Durch die eingesetzte Sonde 6 wird ein Räuminstrument in den Kernbereich eingeführt, durch welches der Hohlraum 5 geschaffen wird und die ausgeschnittenen Gewebeteile entfernt werden.

Als Ersatz für mindestens einen Teil des entfernten Kernbereichs ist eine Zwischenwirbelprothese in Form eines Implantats aus mehreren nacheinander in den Hohlraum 5 einfüllbaren Stützkörpern 7 vorgesehen, die aus einem körperverträglichen elastischen Kunststoff, z.B. Polyurethan, bestehen. Die Stützkörper 7 sind als Rotationskörper, beim dargestellten Beispiel in Form von Kugeln, ausgebildet, deren Abmessungen so gewählt sind, dass sie durch die Sonde 6 in den Hohlraum 5 eingeführt werden können. Die Stützkörper 7 werden, gegebenenfalls mittels eines Stössels 8, in den Hohlraum 5 gestopft, bis dieser durch die sich gegenseitig abstützenden Stützkörper 7 im wesentlichen ausgefüllt ist und die Stützkörper 5 einen zur Uebertragung von Druckkräften geeigneten, neuen Kernbereich der Bandscheibe 3 bilden. Entsprechend den jeweils gegebenen Abmessungen und der Form des zu füllenden Hohlraums 5 und des Querschnitts der Sonde 6 können Anzahl und Abmessungen der Stützkörper 7 beliebig variieren. So ist beispielsweise eine Ausführung möglich, die weniger Stützkörper 7 als die dargestellte Ausführung, z.B. drei in entsprechend passenden Grössen ausgeführte Stützkörper 7, erfordert. Die Stützkörper 7 können mit unterschiedlichen Abmessungen oder, wie dargestellt, mit unter sich gleichen Durchmessern ausgeführt sein. Es ist auch eine Ausführung mit z.B. einlagig angeordneten Stützkörpern 7 möglich, die im Abstand bzw. in Abständen voneinander angeordnet sein können.

Wenn der Hohlraum vollständig gefüllt ist, was z.B. durch einen in Fig. 1 von der rechten Seite her dorsal durch eine Oeffnung 30' in den Hohlraum 5 einführbaren zweiten rohrförmigen Führungsteil, darstellungsgemäss in Form einer Sonde 6' überwacht werden kann, wird die Sonde 6 bzw. werden beide Sonden 6, 6' in bekannter Weise aus dem Ringbereich 4 zurückgezogen, wobei sich der Durchtrittskanal - Oeffnung 30 bzw. 30' - für die Sonde 6 bzw. 6' entsprechend vor dem zuletzt eingefüllten Stützkörper 7 schliesst. Dieser kann auf noch zu beschreibende Weise mit Rückhaltemitteln versehen sein, die ein Austreten durch den Durchtrittskanal erschweren.

Durch die Sonde 6' kann ein Beobachungsinstrument 9 zur Ueberwachung des Ausräumens des Hohlraums 5 und/oder des Implantationsvorganges oder ein nicht dargestelltes Hilfsinstrument zur Unterstützung des Ausräum- bzw. Implantiervorganges eingeführt werden. Es versteht sich, dass anstelle der dargestellten Sonden 6, 6' auch andere geeignete Schutz- und/oder Führungselemente beliebiger Form und Ausführung verwendet werden können.

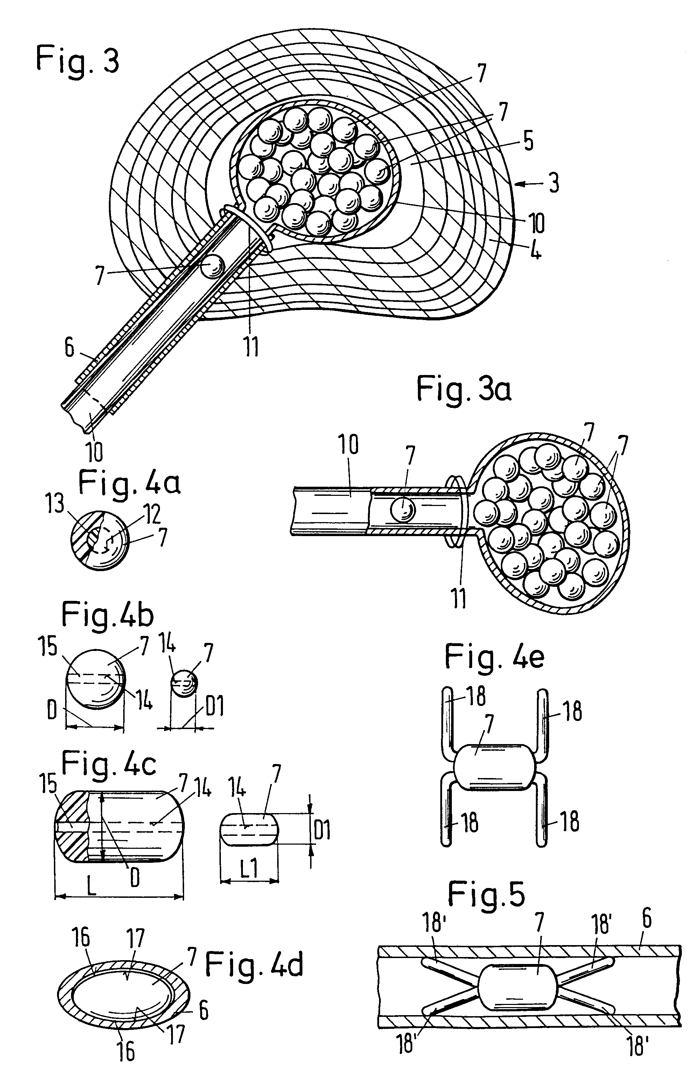

In der Fig. 3 sind die entsprechenden Teile mit gleichen Bezugszeichen versehen. Entsprechend dieser Ausführungsform können die Stützkörper 7 in einer sie umgebenden Umhüllung, darstellungsgemäss in einem Sack 10, angeordnet sein, der leer durch die Sonde 6 in den Hohlraum 5 eingebracht und anschliessend durch die ausserhalb der Sonde 6 verbleibende Oeffnung hindurch mit den Stützkörpern 7 gefüllt wird. Der Sack 10 kann aus einem Gewebe, einem Gestrick oder einer Folie aus einem körperverträglichen, elastischen Kunststoff, z.B. ebenfalls aus Polyurethan, bestehen. Wenn der Sack 10 gefüllt ist und mit den Stützkörpern 7 zu einem kompakten, elastischen, zur Uebertragung von Druckkräften zwischen den Wirbelkörpern 1 und 2 geeigneten Implantat verbunden ist, kann der Sack 10 durch einen Verschlussteil 11, z.B. in Form einer Klammer oder, wie in der Zeichnung angedeutet, einer vorgängig eingeführten Drahtschlinge, abgebunden werden, um die Stützkörper 7 zusammen zu halten. Hierauf wird das Ende des Sacks 10 abgeschnitten und zusammen mit der Sonde 6 zurückgezogen.

Wie insbesondere aus der Fig. 3a hervorgeht, kann sich der Sack 10 entsprechend den jeweils gegebenen anatomischen Verhältnissen an jede beliebige Form des auszufüllenden Hohlraums 5 anpassen.

Es sind zahlreiche Ausführungsformen von Stützkörpern 7 möglich. So sind z.B. anstelle von Rotationskörpern auch Ausführungen mit polyederförmigen Stützkörpern 7 denkbar. Gemäss Fig. 4a können die bei der dargestellten Ausführung kugelförmigen Stützkörper 7 - oder zumindest einer bzw. einige der Stützkörper 7 - je mit einem geschlossenen Hohlraum 12 ausgeführt sein, der einen Einsatz 13 in Form eines kugelförmigen Einschlusses aus einem unter Röntgenbeobachtung sichtbaren Material, z.B. Tantal, als Lageanzeiger für die jeweilige Position der Stützkörper 7 enthalten. Es sind auch Ausführungen ohne Einsatz 13 möglich, wobei durch den Hohlraum 12 eine entsprechend grössere elastische Deformation des Stützkörpers erzielbar ist.

Gemäss Fig. 4b können die oder zumindest einzelne der Stützkörper 7 unterschiedliche Durchmesser D bzw. D1 aufweisen und/oder je mit einem sie durchsetzenden Kanal 14 ausgeführt sein, der offen oder, wie dargestellt, mit einem stabförmigen Einatz 15 als Lageanzeiger versehen sein kann, wobei die jeweilige Orientierung des Einsatzes 15 erkennbar ist.

Gemäss Fig. 4c können Stützkörper 7 mit im wesentlichen zylindrischen, an den Enden abgerundeten Formen ausgeführt sein, die ebenfalls je mit einem Kanal 14 und/oder mit einem stabförmigen Einschluss 15 - oder z.B. mit zwei entsprechenden, in Achsrichtung des Stützkörpers 7 gegeneinander versetzten Einschlüssen - versehen sein können. Auch diese Stützkörper 7 können unterschiedliche Durchmesser D bzw. D1 und/oder unterschiedliche Längen L bzw. L1 aufweisen.

Gemäss Fig. 4d können die oder zumindest einzelne der Stützkörper 7 linsenförmig, beim dargestellten Beispiel in Form eines Ellipsoids, ausgebildet und dementsprechend mit definierten Stützflächen 16 ausgeführt sein, die ein gezieltes Ablegen der Stützkörper 7 mit den gegen die benachbarten Wirbelkörper 1, 2 gerichteten Stützflächen 16 gestatten. Wie aus der Fig. 4d weiter hervorgeht, kann die Sonde 6 mit einem entsprechenden, darstellungsgemäss ovalen, Querschnitt ausgeführt sein, der Führungsbahnen 17 für die Stützflächen 16 bildet.

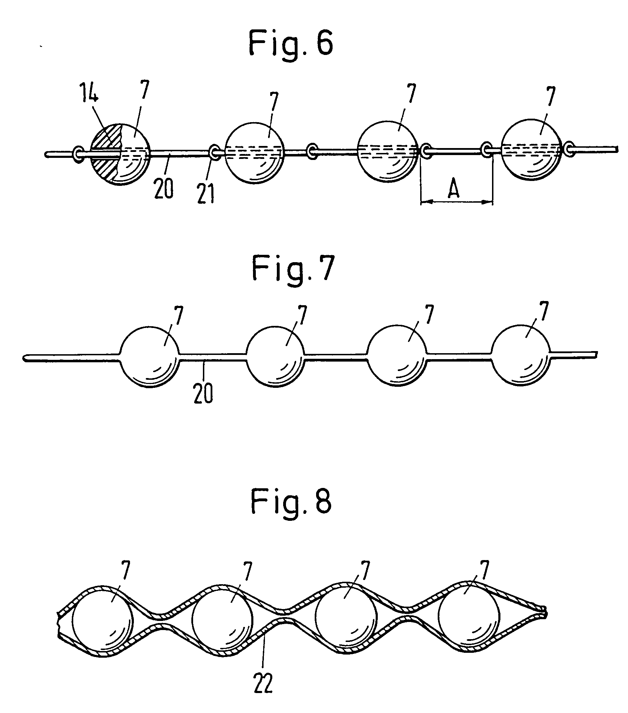

Gemäss Fig. 4e kann mindestens einer der Stützkörper, z.B. der zuletzt in den Hohlraum 5 einzubringende Stützkörper 7, mit mindestens einem, darstellungsgemäss vier elastisch deformierbaren Spreizelementen 18 versehen sein, welche in entspanntem Zustand seitlich vom Stützkörper 7 abstehen und welche, wie in Fig. 5 dargestellt, beim Einführen durch die Sonde 6 in Längsrichtung in gespannte Stellungen 18' deformiert werden und beim Austritt aus der Sonde 6, innerhalb des Hohlraums 5 in den entspannten Zustand zurückfedern und somit einen Austritt aus dem Hohlraum 5 durch die Durchtrittsöffnung für die Sonde 6 verhindern.

Wie aus der Fig. 6 hervorgeht, können mehrere Stützkörper 7 nach Art von Perlen einer Perlenkette auf einem flexiblen, band- oder schnurartigen Träger 20 angeordnet und mit diesem zu einem zusammenhängenden Implantat verbunden sein. Der Träger 20 kann vorzugsweise mit zwischen den Stützkörpern 7 angeordneten Anschlagteilen 21, darstellungsgemäss in Form von am Träger 20 ausgebildeten Knoten, versehen sein, die so angeordnet sind, dass zwischen den Stützkörpern 7 ein vorbestimmter Mindestabstand A eingehalten wird, der zumindest annähernd dem Durchmesser eines der Stützkörper 7 oder - bei Ausführungen mit unterschiedlichen Abmessungen der Stützkörper 7 - der Summe der Radien der einander benachbarten Stützkörper 7 entspricht.

Gemäss Fig. 7 können die Stützkörper 7 und der Träger 20 aus dem gleichen Material gefertigt und zu einem einstückigen Implantat verbunden sein, wobei der Träger 20 als Distanzhalter wirkt. Entsprechend der Darstellung nach Fig. 8 kann eine Anzahl Stützkörper 7 in einer Umhüllung in Form eines die Stützkörper 7 eng umschliessenden Schlauchs 22 angeordnet und mit diesem zu einem zusammenhängenden Implantat verbunden sein. Der Schlauch 22 kann, wie der Sack 10, aus einem entsprechenden Gewebe, einem Gestrick oder einer Folie, gebildet sein.

Zusammenfassend lässt sich somit die Erfindung wie folgt beschreiben:

Als Ersatz für einen nicht mehr tragfähigen Teil des Kernbereichs einer Bandscheibe 3, ist ein Implantat aus mehreren Stützkörpern 7 vorgesehen, welche aus einem elastischen Kunststoff gefertigt sind. Die Stützkörper 7 werden nacheinander mittels einer einen äusseren Ringbereich 4 der Bandscheibe 3 durchsetzenden Sonde 6 in einen im Kernbereich ausgebildeten zentralen Hohlraum 5 eingeführt, bis dieser ausgefüllt ist. Beim Stopfen des Hohlraums 5 mit den Füllkörpern 7 legen sich diese an die Begrenzungswände des Ringbereichs 4 und gegeneinander an und werden unter Belastung elastisch deformiert. Entsprechend wird-ein universell einsetzbares und an Hohlräume 5 beliebiger Form anpassbares Implantat erzielt, welches eine relativ kompakte, elastische Tragstruktur bildet.

## Patentansprüche

1. Zwischenwirbelprothese mit einem in einen zentralen Hohlraum (5) eines Kernbereichs einer Bandscheibe (3) einsetzbaren, zur Übertragung von Druckkräften geeigneten Implantat als Ersatz für einen Teil dieses Kernbereichs, dadurch gekennzeichnet, dass das Implantat mindestens drei in den zentralen Hohlraum (5) einfüllbare und in diesem positionierbare, elastisch verformbare Stützkörper (7) enthält.

2. Prothese nach Anspruch 1, dadurch gekennzeichnet, dass die Stützkörper (7) aus einem elastischen Kunststoff gefertigt sind.

3. Prothese nach Anspruch 1 oder 2, dadurch gekennzeichnet, dass die Stützkörper (7) in Form von Rotationskörpern ausgeführt sind.

4. Prothese nach einem der vorangehenden Ansprüche, dadurch gekennzeichnet, dass die eingefüllten Stützkörper (7) mit unterschiedlichen Abmessungen (D, D1, L, L1) ausgeführt sind.

5. Prothese nach einem der vorangehenden Ansprüche, dadurch gekennzeichnet, dass mindestens einer der Stützkörper (7) mit einem ihn durchsetzenden Kanal (14) ausgeführt ist.

6. Prothese nach einem der vorangehenden Ansprüche, dadurch gekennzeichnet, dass mindestens einer der Stützkörper (7) mit einem geschlossenen Hohlraum (12) ausgeführt ist.

7. Prothese nach einem der vorangehenden Ansprüche, dadurch gekennzeichnet, dass mindestens einer der Stützkörper (7) mit mindestens einem Einsatz (13, 15) aus einem unter Röntgenbeobachtung sichtbaren Material, z.B. Tantal, ausgeführt ist.

8. Prothese nach einem der vorangehenden Ansprüche, dadurch gekennzeichnet, dass die Stützkörper (7) nach Art einer Perlenkette auf einem flexiblen, z.B. schnurartigen Träger (20) angeordnet sind, der Haltemittel zum Einhalten eines vorbestimmten Mindestabstandes (A) zwischen den Stützkörpern (7) aufweist.

9. Prothese nach Anspruch 8, dadurch gekennzeichnet, dass der Mindestabstand (A) zwischen den Stützkörpern (7) zumindest annähernd dem Durchmesser (D) eines der Stützkörper (7) entspricht.

10. Prothese nach Anspruch 8 oder 9, dadurch gekennzeichnet, dass die Haltemittel mindestens einen zwischen den Stützkörpern (7) angebrachten Anschlagteil (21), z.B. in Form eines im flexiblen Träger (20) ausgebildeten Knotens, enthalten.

11. Prothese nach Anspruch 8 oder 9, dadurch gekennzeichnet, dass die Stützkörper (7) und der flexible Träger (20) aus dem gleichen Material gefertigt und zu einem einstückigen Implantat verbunden sind.

12. Prothese nach einem der vorangehenden Ansprüche, dadurch gekennzeichnet, dass die Stützkörper (7) in einer sie umgebenden Umhüllung (10, 22) angeordnet sind.

13. Prothese nach einem der vorangehenden Ansprüche, dadurch gekennzeichnet, dass mindestens einer der Stützkörper (7) mit mindestens einem von einem Hauptteil des Stützkörpers seitlich abstehenden, elastisch verformbaren Spreizelement (18) versehen ist.

14. Prothese nach einem der vorangehenden Ansprüche, dadurch gekennzeichnet, dass mindestens einer der Stützkörper (7) eine definierte Stützfläche (16) aufweist, welche zum Zusammenwirken mit einer in einem Führungsteil (Sonde 6) ausgebildeten Führungsbahn (17) bestimmt ist und durch welche der Stützkörper (7) in einer definierten Lage im zentralen Hohlraum (5) positionierbar ist.

15. Verfahren zum Implantieren einer Zwischenwirbelprothese, die ein in einen Kernbereich einer Bandscheibe (3) einsetzbares Implantat mit mindestens drei elastisch verformbaren Stützkörpern (7) enthält, dadurch gekennzeichnet, dass
- in einem ersten Schritt in einem äusseren Ringbereich (4) der Bandscheibe (3) eine diesen gegen den Kernbereich hin durchsetzende Oeffnung (30) angebracht wird,
- in einem folgenden Schritt ein Räuminstrument durch die Oeffnung (30) gegen den Kernbereich eingeführt und in diesem durch Herausschneiden von nicht mehr tragfähigen Gewebeteilen des Bandscheibenkerns ein zentraler Hohlraum (5) geschaffen wird und die ausgeschnittenen Gewebeteile durch die Oeffnung (30) hindurch entfernt werden,
- in einem weiteren Schritt die Stützkörper (7) durch die Oeffnung (30) in den Hohlraum (5) eingeführt werden, wobei der Hohlraum (5) im wesentlichen ausgefüllt wird und die Stützkörper (7) eine druckfeste Tragstruktur bilden, und
- in einem folgenden Schritt, nach dem Einführen des letzten Stützkörpers (7), die Oeffnung (30) verschlossen wird.

16. Verfahren nach Anspruch 15, dadurch gekennzeichnet, dass in die den äusseren Ringbereich (4) durchsetzende Oeffnung (30) ein rohrförmiger Schutz- und/oder Führungsteil (Sonde 6) eingesetzt wird, durch den das Räuminstrument und die Stützkörper (7) in den Kernbereich eingeführt werden.

17. Verfahren nach Anspruch 15 oder 16, dadurch gekennzeichnet, dass im äusseren Ringbereich (4) eine zweite Oeffnung (30') angebracht wird, und dass durch diese ein zweiter Führungsteil (Sonde 6') gegen den Kernbereich eingeführt wird, durch welchen das Ausräumen des Hohlraumes (5) und/oder das Einführen der Stützkörper (7) überwacht bzw. beeinflusst werden kann.

18. Verfahren nach einem der Ansprüche 15 bis 17, dadurch gekennzeichnet, dass nach dem Ausräumen des zentralen Hohlraumes (5) eine Umhüllung (10) für die später einzuführenden Stützkörper (7) in den Hohlraum (5) eingebracht wird, wobei eine Einfüllöffnung ausserhalb des äusseren Ringbereichs (4) verbleibt.
